# EUROPEAN PATENT APPLICATION

(11) **EP 3 437 683 A1**
(43) Date of publication of application: **06.02.2019**
(21) Application number: 17774242.6
(22) Date of filing: 13.03.2017
(51) Int. Cl.: A61M 5/44, A61B 6/00

(54) **CONTRAST MEDIUM TEMPERATURE-MAINTAINING AND INJECTING SYSTEM FOR MICROCATHETER, AND THERAPEUTIC SUSPENSION MEDICINE TEMPERATURE-MAINTAINING AND INJECTING SYSTEM**

(30) Priority: 01.04.2016 JP 2016083200
(71) Applicant: Hori, Atsushi, Sakai-shi, Osaka 592-8344 (JP); Hori, Shinichi, Sakai-shi, Osaka 592-8343 (JP); Mizoguchi, Kazuaki, Okayama-shi, Okayama 704-8174 (JP); Cosmic M.E.Inc., Kawaguchi-shi, Saitama 333-0848 (JP); APOLLO RT CO. LTD., Kowloon, Hong Kong (CN)
(72) Inventor: Hori, Atsushi, Sakai-shi, Osaka 592-8344 (JP); Hori, Shinichi, Sakai-shi, Osaka 592-8343 (JP); Mizoguchi, Kazuaki, Okayama-shi, Okayama 704-8174 (JP); Cosmic M.E.Inc., Kawaguchi-shi, Saitama 333-0848 (JP); APOLLO RT CO. LTD., Kowloon, Hong Kong (CN)
(74) Representative: Herzog, Fiesser & Partner Patentanwälte PartG mbB
(86) International application number: PCT/JP2017/010026
(87) International publication number: WO 2017/169698

(57) **Abstract**

An object of the present invention is to provide a system for temperature-maintaining and injecting a contrast medium allowing an injection pressure of an injecting contrast medium to be reduced and assuring easy injection of the contrast medium. The system (100) for temperature-maintaining and injecting the contrast medium of the present invention is characterized by comprising an injector (I), a pump control device (10f), a pump head portion (10a), a small diameter syringe (30), and an outer peripheral temperature-maintaining heater unit (10c) to be mounted on an outer periphery of the small diameter syringe (30), and the outer peripheral temperature-maintaining heater unit (10c) is connected to a heat source output terminal provided on the pump head portion (10a).

## Description

### TECHNICAL FIELD

The present invention relates to a system for temperature-maintaining and injecting contrast medium for a microcatheter and a system for temperature-maintaining and injecting therapeutic suspension medicine for a microcatheter.

### BACKGROUND ART

So far a medical practice for moving a catheter percutaneously inserted in a blood vessel to brain, heart or internal organs such as abdominal region for administering and injection of a therapeutic agent, an embolic substance, a contrast medium or the like thereinto has been performed. Recently with advances in medicine, injection of a therapeutic agent, an embolic substance, a contrast medium or the like into a thinner peripheral blood vessel became necessary, and research and development relating to enhancement of functionality of a microcatheter that can be inserted into such a thin peripheral blood vessel are continued enthusiastically.

In the research and development on a microcatheter, various requirements for operability of the microcatheter are demanded since it is necessary for an operator to advance the microcatheter surely along the tortuous and thinner peripheral blood vessel. Such requirements for functionality include pushability for surely transmitting a pushing force from an operator to a tip of the microcatheter, torque transmission property for surely transmitting a turning force applied by an operator to a tip of the microcatheter, followability to a guide wire for advancing in a winding blood vessel along the guide wire passing through an inner cavity of the catheter, and kink resistance of the microcatheter causing no bending even at a bent portion or a curved portion of a blood vessel. In order to achieve such operability, configuration for providing a reinforcing layer having a braided structure or a coil structure is employed on many microcatheters (see, for example, Patent Documents 1 to 3).

### PRIOR ART DOCUMENTS

### Patent Documents

Patent Document 1: JP 2008-295825 A
Patent Document 2: JP 2009-089814 A
Patent Document 3: JP 2014-113485 A

### Non-Patent Documents

Non-Patent Document 1: Magazine of Tohoku Sectional Meeting of Japanese Society of Radiological Technology, No. 13, January 2004, 41st Tohoku Sectional Annual Meeting Publication, Page 64 "Study on Optimum Iodine Concentration of Contrast Medium used on Microcatheter"
Non-Patent Document 2: Kora Shinichi et al. Journal of New Remedies and Clinics", Vol. 61, No. 5, pp. 76-82, 2012

### SUMMARY OF THE INVENTION

### PROBLEM TO BE SOLVED BY THE INVENTION

The diameter of an inner cavity of a microcatheter becomes thinner, and in addition, its effective length becomes longer (for example, an effective length becomes longer from 125 cm to 150 cm), resulting in further burden being imposed on injection. Under such a situation, for example, in either of a case of a contrast examination for imaging of a site to be treated or a case of treatment of embolus of the affected part defined by a contrast examination, there arises a problem with high viscosity injection (i.e. a high injection pressure is required) when injecting a contrast medium or a therapeutic suspension medicine for embolus.

In the contrast examination, for example in case of injection of a contrast medium by pushing a syringe by hand, an injection pressure and rate of a contrast medium cannot be maintained constant, and in case of injection of a contrast medium by means of a usual high pressure injector (100 to 150 cc of a syringe size), an accuracy of injection cannot be kept constant. Further, in injection of a therapeutic suspension for embolus including an anticancer drug, injection becomes difficult due to a viscosity of the suspension and a smaller diameter of a microcatheter.

However, in a contrast examination, trials for solving the above-mentioned problems and assisting the solution of the problems has been hardly made, and only a method of enabling an injection amount to be increased to some extent by reduction of a contrast medium viscosity by heating and matching for optimum concentration of a contrast medium is disclosed in the above-mentioned Non-Patent Document 1 recently opened to the public (see Non-Patent Document 1). Similarly, also for treatment of embolus, trials for solving the above-mentioned problem has hardly been made so far, and only heating of a mixing vessel containing an anticancer drug and a contrast medium in a water bath as disclosed in Non-Patent Document 2 has been employed.

Therefore, an object of the present invention is to provide a system for temperature-maintaining and injecting a contrast medium for a microcatheter and a system for temperature-maintaining and injecting a therapeutic suspension medicine for a microcatheter which allow an injection pressure of a contrast medium and a therapeutic suspension medicine for embolus to be reduced and make easy injection of a contrast medium and a therapeutic suspension medicine for embolus.

### MEANS TO SOLVE THE PROBLEM

The system for temperature-maintaining and injecting a contrast medium for a microcatheter comprises an injector for injecting the contrast medium and a pump control device connected to the injector, wherein the injector for injecting the contrast medium comprises a pump head portion and a small diameter syringe connected to the pump head portion, wherein the system for temperature-maintaining and injecting the contrast medium further comprises an outer peripheral temperature-maintaining heater unit to be attached on an outer periphery of the small diameter syringe, and wherein the outer peripheral temperature-maintaining heater unit to be attached on the outer periphery of the small diameter syringe is connected to a heat source output terminal provided on the pump head portion.

The system for temperature-maintaining and injecting a therapeutic suspension medicine for a microcatheter comprises a microcatheter, a syringe for manual operation for containing a suspension including an anticancer drug and a contrast medium for treatment of embolus, and a connecting portion for connecting the syringe for manual operation and the microcatheter, wherein the system for temperature-maintaining and injecting a therapeutic suspension medicine further comprises an outer peripheral temperature-maintaining heater unit being mounted on an outer periphery of the syringe for manual operation.

### EFFECTS OF THE INVENTION

The system for temperature-maintaining and injecting a contrast medium for a microcatheter and the system for temperature-maintaining and injecting a therapeutic suspension medicine for a microcatheter of the present invention are capable of reducing viscosities of a contrast medium and a therapeutic suspension medicine for embolus (reducing an injection pressure) and making the injection easy.

### BRIEF DESCRIPTION OF THE DRAWINGS

FIG. 1 is a block diagram schematically showing a system for temperature-maintaining and injecting a contrast medium for a microcatheter according to one embodiment of the present invention.
FIG. 2 is a view showing a combined state of a pump head portion and a small diameter syringe to be used on the system for temperature-maintaining and injecting the contrast medium of FIG. 1.
FIG. 3(A) is a view showing a connected state of a long plunger being connected to a small diameter syringe, and FIG. 3(B) is a view showing a separated state of the long plunger being separated from the small diameter syringe.
FIG. 4 is a schematic view showing an outer peripheral temperature-maintaining heater unit being mounted on a small diameter syringe and a contrast medium bottle.
FIG. 5(A) is a reference drawing showing a non-separable plunger being connected to the pump head portion, and FIG. 5(B) is a view showing a connected state where a plunger from which the long plunger is detached is connected to the pump head portion in one embodiment of the present invention.
FIG. 6(A) is a reference drawing showing a fixed state of pump head portion of a general contrast medium injector being fixed to a catheter operating table, and FIG. 6(B) is a view showing an attached state of pump head portion being mounted on an arm stand in one embodiment of the present invention.
FIG. 7(A) is a view showing a pump head portion having a built-in drive unit according to one embodiment of the present invention, and FIG. 7(B) is a view showing a pump head portion to which an external drive unit can be mounted according to another embodiment of the present invention, wherein the drive unit is not built in the pump head portion.
FIG. 8 is a graph showing a comparison of an effect in use of a film heater for a contrast medium bottle and non-use thereof in a temperature maintaining experiment.
FIG. 9 is a view showing an example of a hand switch portion.
FIG. 10 is a schematic view showing the system for temperature-maintaining and injecting therapeutic suspension medicine according to one embodiment of the present invention.
FIG. 11 is a view showing a state where an outer peripheral temperature-maintaining heater unit for a small diameter syringe and an outer peripheral temperature-maintaining heater unit for a syringe for manual operation are connected to the pump head portion.
FIG. 12 is a view showing a film heater for a small diameter syringe or a syringe for manual operation.
FIG. 13 is a view showing a film heater for a contrast medium bottle.

### EMBODIMENT FOR CARRYING OUT THE INVENTION

### <First embodiment>

By referring to the attached drawings, an embodiment of a system for temperature-maintaining and injecting a contrast medium for a microcatheter of the present invention is described below. It is noted that the following embodiment is only an example, and the system for temperature-maintaining and injecting the contrast medium of the present invention is not limited to the following embodiment.

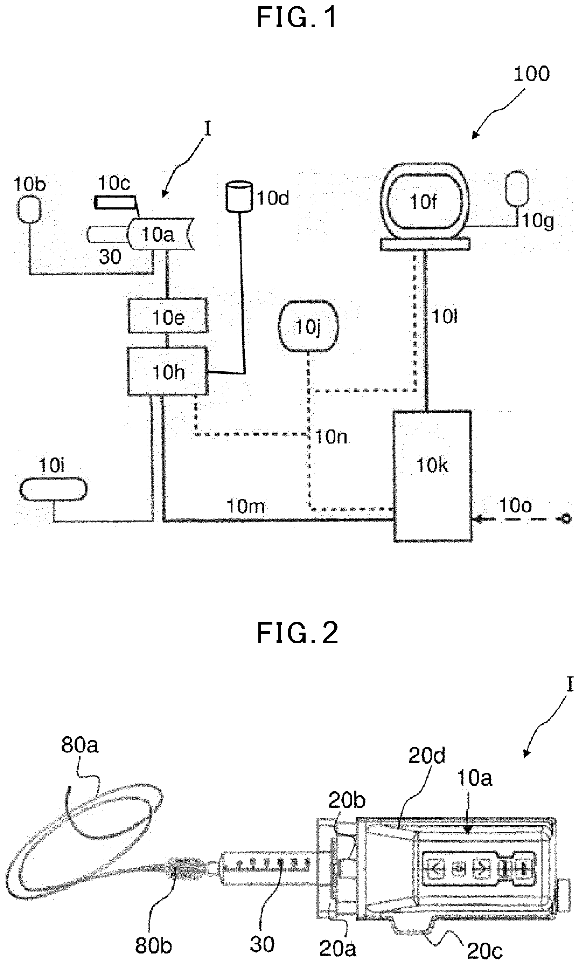

As shown in a block diagram of FIG. 1, the system for temperature-maintaining and injecting contrast medium (hereinafter simply referred to as an injecting system) 100 has a movable pump head portion 10a. The movable pump head portion 10a is arranged on an injection site of a patient, and injection of a contrast medium is initiated by means of any of a hand switch portion 10b and a foot switch portion 10i provided at an operating field or a hand switch portion 10g provided in an operation room according to an injection rate and amount to be set by an injection control section 10f (pump control device).

The injecting system 100 according to one embodiment of the present invention is described below in detail using FIGS. 1 to 9.

FIG. 1 is a block diagram schematically showing the injecting system according to one embodiment of the present invention, and FIG. 2 is a view showing a combined state of a pump head portion and a small diameter syringe to be used on the injecting system of FIG. 1.

In FIG. 1, a reference symbol 100 represents the whole configuration of the injecting system, 10f represents an injection control section (pump control device), 10b and 10i represent a hand switch portion and a foot switch portion, respectively provided at an operating field, 10g represents a hand switch portion provided in an operation room, 10a represents a movable pump head portion, and 30 represents a small diameter syringe to be mounted on the pump head portion 10a. Further, a reference symbol 10k represents a main unit as a main power source, 10h represents a relay box disposed between the main unit 10k and the pump head portion 10a, 10e represents a motor box disposed between the relay box 10h and the pump head portion 10a, 10c represents an outer peripheral temperature-maintaining heater unit for a small diameter syringe, 10d represents an outer peripheral temperature-maintaining heater unit of a syringe for manual operation, and 10j represents a monitor provided for an operator. Reference symbols 101, 10m, 10n and 10o are cables for power supply or cables for signal transmission connecting the above-mentioned components.

FIG. 2 is a view showing a combined state of the pump head portion and the small diameter syringe to be used on the injecting system of FIG. 1. In FIG. 2, a reference symbol 20a represents a syringe mounting portion, 20b represents a plunger connecting portion, 20c represents a heat source output terminal, 20d represents a housing, 80b represents a wing portion for fixing a microcatheter to a syringe, and 80a represents a microcatheter.

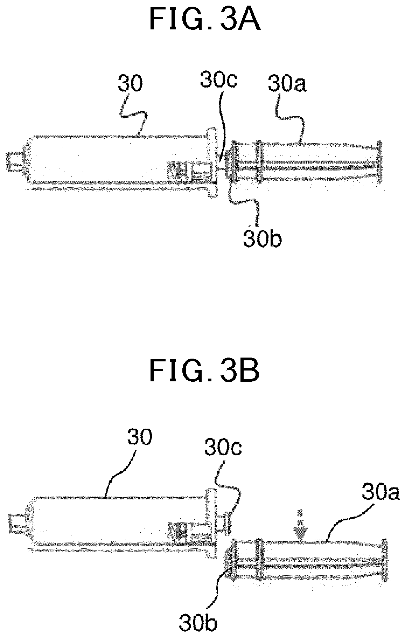

FIG. 3(A) is a view showing a connected state of a long plunger being connected to a small diameter syringe, and FIG. 3(B) is a view showing a separated state of the long plunger being separated from the small diameter syringe. In FIG. 3, a reference symbol 30a represents a separable long plunger, 30c represents a short plunger to be connected to a gasket of the short syringe 30, and 30b represents a long plunger connecting portion for connecting the long plunger 30a to the short plunger 30c. FIG. 3(A) shows that the separable long plunger 30a is connected to the small diameter syringe 30 by connecting the long plunger 30a to the short plunger 30c with the long plunger connecting portion 30b. Further, FIG. 3(B) shows that the separable long plunger 30a is separated from the short plunger 30c of the small diameter syringe 30 and the small diameter syringe 30 has only the short plunger 30c.

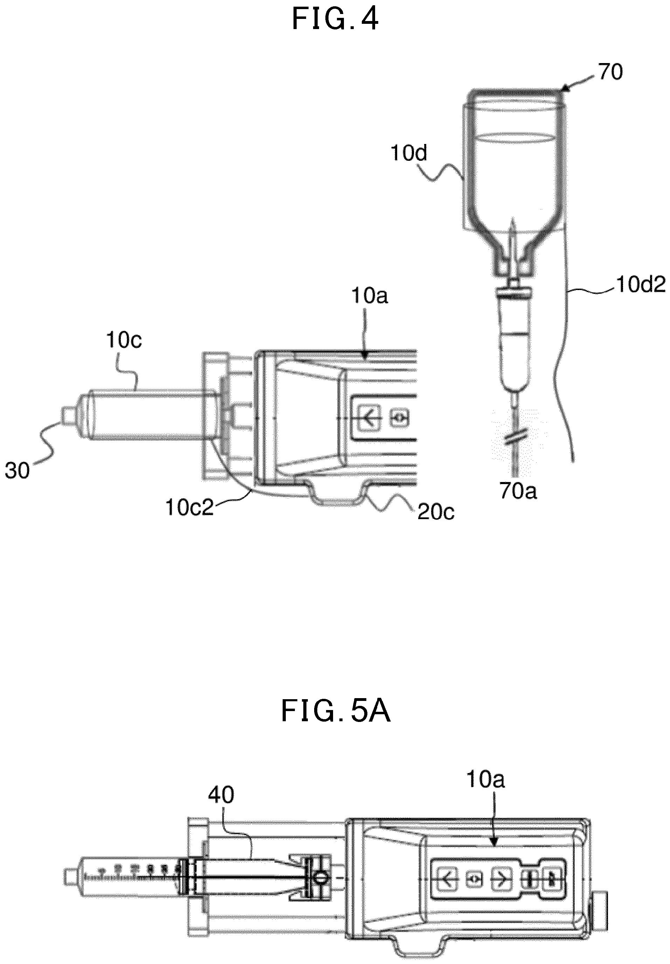

FIG. 4 is a schematic view showing an outer peripheral temperature-maintaining heater unit being mounted on a small diameter syringe and a contrast medium bottle. 10c represents the outer peripheral temperature-maintaining heater unit for a small diameter syringe, 10d represents the outer peripheral temperature-maintaining heater unit for the contrast medium bottle. 10c2 and 10d2 represent power cords for heat source to the outer peripheral temperature-maintaining heater units 10c, 10d, respectively. Further a reference symbol 70 represents the contrast medium bottle, and 70a represents a contrast medium filling line.

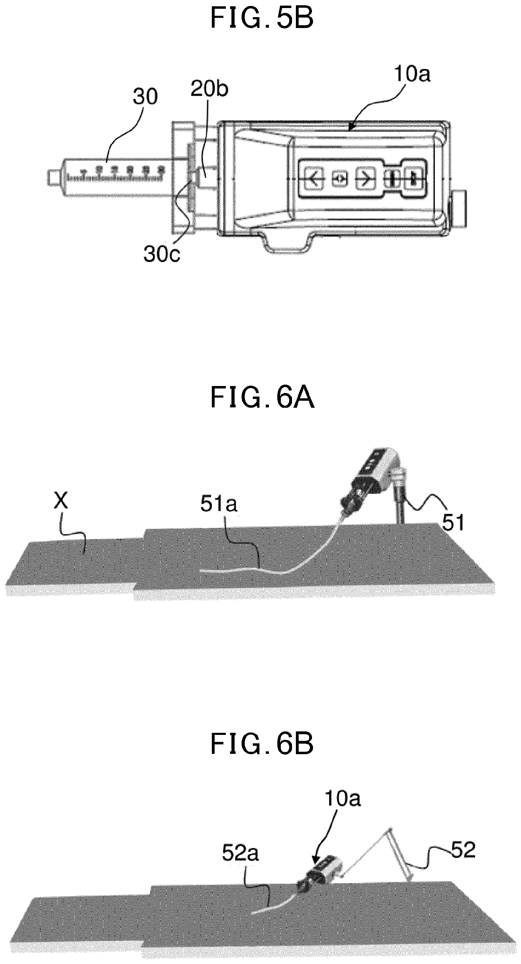

FIG. 5(A) is a reference drawing showing a non-separable plunger being connected to the pump head portion, and FIG. 5(B) is a view showing a connected state where a plunger from which the long plunger is detached is connected to the pump head portion in one embodiment of the present invention. In FIG. 5(A), a reference symbol 40 represents a plunger to be used on a general non-separable syringe.

FIG. 6(A) is a reference drawing showing a fixed state of a pump head portion of a general contrast medium injector being fixed to a catheter operating table, and FIG. 6(B) is a view showing an attached state of a pump head portion being mounted on an arm stand in one embodiment of the present invention. In FIG. 6(A), a reference symbol X represents a catheter operating table to be used under X-ray environment, 51 represents a fixing pole mounted on a side rail of the catheter operating table X for fixing a pump head of a general contrast medium injector, and 51a represents a long extended injection tube extending from the pump head mounted on the fixed pole 51 to an operating field. In FIG. 6(B), a reference symbol 52 represents an arm stand for attaching the pump head portion 10a in this embodiment, and 52a represents a short extended injection tube slightly extending in a short length from the pump head 10a moved to the vicinity of the operating field by the arm stand 52.

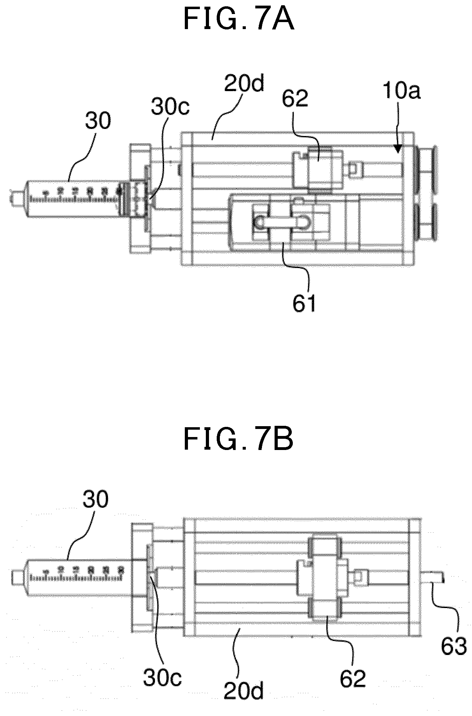

FIG. 7(A) is a view showing a pump head portion having a built-in drive unit according to one embodiment of the present invention, and FIG. 7(B) is a view showing a pump head portion to which an external drive unit can be mounted according to another embodiment of the present invention, wherein the drive unit is not built in the pump head portion. In FIG. 7(A) and FIG. 7(B), a reference symbol 62 represents a plunger drive unit moving so as to move the plunger (the short plunger 30c) of the small diameter syringe 30, and 61 represents a motor drive unit for allowing the plunger drive unit 62 to be moved by a motor. Further in FIG 7(B), a reference symbol 62 represents the plunger drive unit similarly to FIG. 7(A), and 63 shows a part of a flexible shaft (tube) for moving the plunger drive unit 62 by transmitting power of an external motor not shown in the drawing.

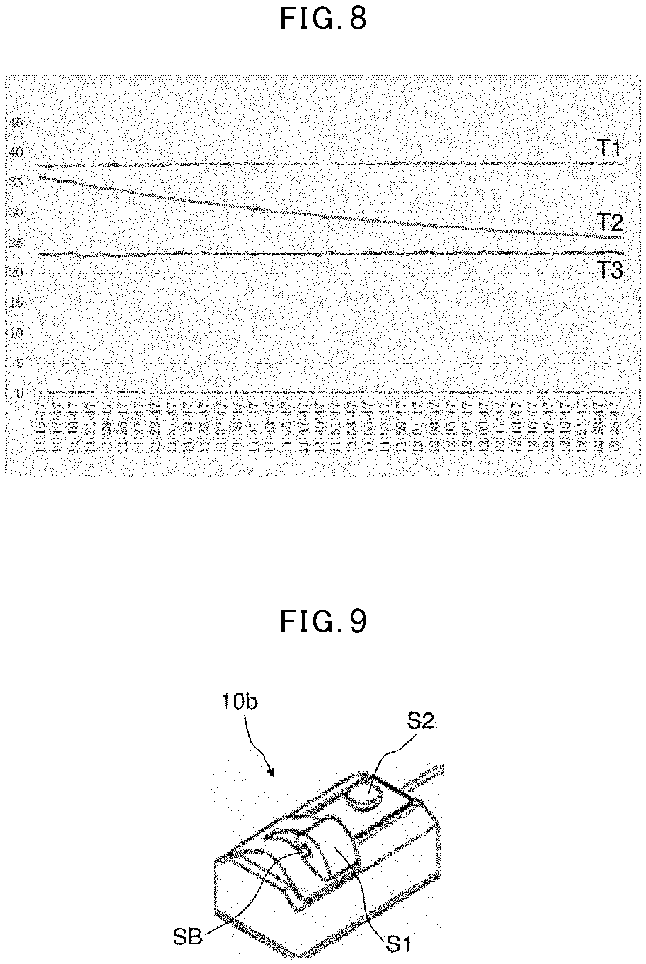

FIG. 8 is a graph showing a comparison of an effect in use of a film heater for a contrast medium bottle and non-use thereof in a temperature maintaining experiment. In FIG. 8, an axis of abscissa indicates a time, and an axis of ordinate indicates a temperature (°C). In FIG. 8, T1 represents a temperature of the outer periphery of the contrast medium bottle in an elapsed time when using the outer peripheral temperature-maintaining heater unit 10d for a contrast medium bottle, T2 represents a temperature of the outer periphery of the contrast medium bottle in an elapsed time when using no outer peripheral temperature-maintaining heater unit 10d, and T3 represents an ambient temperature.

As shown in FIG. 1, the injecting system 100 according to this embodiment comprises the contrast medium injector I and the injection control section (pump control device) 10f.

The injecting system 100 is used for injecting a contrast medium into a patient for catheter treatment. In this embodiment, as shown in FIG. 1, in addition to the injector I and the injection control section 10f, the injecting system 100 comprises the switch portions (a hand switch portion 10b provided at an operating field, the foot switch portion 10i provided at an operating field and the hand switch portion 10g provided in an operation room) for operation of contrast medium injection by the injector I, the main unit 10k as a main power source, the relay box 10h connected to the main unit 10k, etc. Further, the injecting system 100 includes the catheter operating table X (see FIG. 6), which is provided in an operation room, for treatment of a patient and a suspension stand (not shown) for suspending the contrast medium bottle 70 (see FIG. 4).

The injecting system 100 injects a contrast medium into the interior of the body of a patient by operating any of the switch portions 10b, 10i, 10g, thereby motor-driving the injector I. More specifically, a contrast medium in the small diameter syringe 30 is injected into the interior of the body of a patient through the microcatheter 80a (see FIG. 2) connected to the injector I. The switch portions may be operated not only for injecting a contrast medium but also for filling a contrast medium into the small diameter syringe 30. For example, as shown in FIG. 9, the switch portion 10b provided at an operating field may include an injection operation switch S1 and a filling operation switch S2. The injection operation switch S1 has a safety button SB, and when the safety button SB is pressed, the lever-shaped injection operation switch S1 can be operated, which can prevent an erroneous operation of the switch. When the injection operation switch S1 is operated, a predetermined amount of a contrast medium is injected at a set injection rate. When the predetermined amount of the contrast medium has been injected or when the operation of the injection operation switch S1 is stopped, the injection of the contrast medium is terminated. When the filling operation switch S2 is operated, filling of the contrast medium into the small diameter syringe 30 is carried out automatically at a set filling rate. When the small diameter syringe 30 is fully filled with the contrast medium or when the filling operation switch S2 is pressed again, the filling is terminated. It is noted that the above-mentioned configuration of the injecting system 100 is an example, and the configuration of the injecting system 100 is not limited to that shown in FIG. 1. In FIG. 2, while the microcatheter 80a is connected directly to the injector I (the small diameter syringe 30), the microcatheter 80a may be connected indirectly to the injector I via the short extended injection tube 52a having a necessary minimum length, or the like.

As shown in FIGS. 1 and 2, the injector I has the pump head portion 10a and the small diameter syringe 30 connected to the pump head portion 10a.

The pump head portion 10a is an injector head for injecting the contrast medium into a patient by sliding the plunger of the small diameter syringe 30. As shown in FIG. 2, the pump head portion 10a according to this embodiment comprises a housing 20d, a syringe mounting portion 20a provided on the housing 20d and to be connected to the small diameter syringe 30, and a plunger connecting portion 20b to which the plunger (the short plunger 30c) of the small diameter syringe 30 is connected.

In this embodiment, the syringe mounting portion 20a is designed as a single type holding a single small diameter syringe 30. The structure of the syringe mounting portion 20a is not limited particularly as far as the small diameter syringe 30 can be mounted. For example, the syringe mounting portion 20a can be configured to be engaged with a flange portion of the small dimeter syringe 30 to lock the syringe. The structure of the plunger connecting portion 20b is not limited particularly as far as the short plunger 30c of the small diameter syringe 30 can be connected to the pump head portion 10a.

As shown in FIG. 2 and FIG. 4, the pump head portion 10a has the heat source output terminal 20c to which the power cord 10c2 for heat source is connected in order to heat the outer peripheral temperature-maintaining heater unit 10c. While the position where the heat source output terminal 20c is provided is not limited particularly, in this embodiment, the heat source output terminal 20c is disposed on a side wall of the pump head portion 10a in the vicinity of the connecting part which connects the pump head portion 10a with the small diameter syringe. The power cord 10c2 for heat source has an insertion type connecting part to be inserted into the heat source output terminal 20c. Therefore, the power cord 10c2 for heat source can be easily connected to the heat source output terminal 20c in parallel with the connection of the plunger of the small diameter syringe 30 to the plunger connecting portion 20b. Further, by connecting the power cord 10c2 for heat source to the heat source output terminal 20c of the pump head portion 10a, the power cord 10c2 for heat source will not disturb manipulation of catheter. Namely, when the pump head portion 10a and the catheter operating table X are moved during the treatment, the power cord 10c2 for heat source moves together with the pump head portion 10a and the catheter operating table X, and there is no need of considering an excessive length of the power cord 10c2 for heat source. Therefore, a situation such that the power cord 10c2 for heat source is located at a position disturbing manipulation of catheter treatment as a result of movement of the pump head portion 10a and the catheter operating table X does not arise unlike the case where the power cord 10c2 for heat source is connected to a position other than the catheter operating table X (for example, the main unit 10k).

The pump head portion 10a is designed as a small size pump head portion holding a single small diameter syringe 30. It is preferable that the pump head portion 10a is a small size pump head portion for a syringe having a volume of 50 cc or less. The small size pump head portion 10a can be one having, for example, a weight of 500 to 3,000 g, a width (a length in a direction vertical to the axial direction of the small diameter syringe 30) of 50 to 150 mm, and a length (a length in the axial direction of the small diameter syringe 30) of 150 to 250 mm (in a shrunk state without syringe).

Further, as shown in FIGS. 7(A) and 7(B), the pump head portion 10a is provided with the plunger drive unit 62 for sliding the plunger of the small diameter syringe 30. As shown in FIG. 7(A), the pump head portion 10a may be provided with the motor drive unit 61. Further, as shown in FIG. 7(B), the plunger drive unit 62 may be designed so as to be connected, by means of a flexible shaft 63, to a motor drive unit (not shown) provided separately for operating the pump head portion 10a, thereby being driven by a driving force of the motor drive unit. As shown in FIG. 7(B), in the case of the plunger drive unit 62 being driven by the flexible shaft 63, it is possible to make a weight of the pump head portion 10 lighter and a size thereof smaller.

Further, as shown in FIG. 6(B), the injecting system 100 according to this embodiment comprises an arm stand 52 having the pump head portion 10a mounted thereon and being movable to an operating field. It is noted that herein "movable to an operating field" means that the pump head portion 10a can be moved to a position where the small diameter syringe 30 can be connected directly to the microcatheter 80a or a position in the vicinity of the wing portion 80b of the microcatheter 80a (for example, a necessary length of an extended injection tube of 500 mm or less, more preferably 250 mm or less). In this embodiment, as shown in FIG. 6(B), the arm stand 52 is shown as a horizontal-vertical arm stand, in which a plurality of arms (two arms in FIG. 6(B)) is link-connected, and a position of the pump head portion 10a in a vertical direction and a horizontal direction can be adjusted. It is noted that the arm stand 52 is preferably designed as a thin arm so as not to obstruct a visual field in an operating field.

Further, in this embodiment, the arm stand 52 is mounted on the catheter operating table X, and the pump head portion 10a can be moved toward the operating field by moving the arm stand 52 on the catheter operating table X. The arm stand 52 is detachably mounted on a side rail provided at a side portion of the catheter operating table X with a bracket or the like, and moves so as to be capable of adjusting its position in a vertical direction and a horizontal direction not to obstruct catheter treatment of an operator. It is preferable that the arm stand 52 is of light-weight and simple configuration so that it can be mounted on the catheter operating table X while holding the pump head portion 10a and the small diameter syringe 30. It is noted that the structure of the arm stand 52 is not limited particularly as far as it can be mounted on the catheter operating table X and moved to an operating field or the vicinity of the operating field.

In catheter treatment, catheter operation is carried out while continuously moving the catheter operating table X in order to match the position of X-ray irradiation to the affected part of a patient. Therefore, the pump head portion 10a and the small diameter syringe 30 moved together with the catheter operating table X by mounting the arm stand 52 on the catheter operating table X. Accordingly, operability of the catheter is enhanced, and it is not necessary to provide an excessive length of the extended injection tube in consideration of movement of the catheter operating table X, resulting in disuse of the extended injection tube or exceedingly shortening of the length thereof easily.

As shown in FIGS. 3(A) and 3(B), the small diameter syringe 30 to be connected to the pump head portion 10a comprises a tubular cylinder member and a plunger (in the embodiment, a long plunger 30a, a short plunger 30c) slidably inserted into the cylinder member.

In this embodiment, the small diameter syringe 30, for example, having a volume of 30 to 50 cc and an inner diameter of 20 to 30 mm is used. It is more preferable that the volume of the small diameter syringe 30 is set to be 30 cc calculated from a maximum amount for one injection of a contrast medium in microcatheter treatment. This maximum amount for one injection can be obtained from, for example, an actual example of a series of imaging methods (angiography, CT imaging) required for embolotherapy of hepatic arteries.

As mentioned above, by using the small diameter syringe 30, a small size and light-weight injector I is obtained after the small diameter syringe 30 is mounted on the pump head portion 10a. Thus, the pump head portion 10a and the small diameter syringe 30 can be moved easily to an operating field or to the vicinity of the operating field with the arm stand 52. Therefore, a large size system such as a system using a relatively large ceiling-suspension type pump head is not necessary, and introduction cost for the injecting system 100 can be reduced. Further, by use of the small diameter syringe 30, reduction of an injection pressure as well as enhancement of operability can be achieved.

In this embodiment, as shown in FIGS. 3(A) and 3(B), the small diameter syringe 30 comprises the short plunger 30c to be connected to the gasket of the small diameter syringe 30 and the long plunger 30a to be connected to the short plunger 30c. The long plunger 30a is connected to the short plunger 30c with the long plunger connecting portion 30b. The method of connecting the short plunger 30c to the long plunger 30a is not limited particularly, and these are connected, for example, by engaging. In this embodiment, the small diameter syringe 30 can be in a connected state or in a separated state by sliding the long plunger 30a relative to the short plunger 30c in a direction vertical to the axial direction of the small diameter syringe 30.

When filling a contrast medium in the small diameter syringe 30 by hand, the filling of a contrast medium in the small diameter syringe 30 by hand becomes easy in the connected state in which the long plunger 30a is connected to the short plunger 30c. When connecting the small diameter syringe 30 to the pump head portion 10a, the long plunger 30a may be formed in the separated state of being separated from the short plunger 30c. As a result, filling by hand becomes easy, and in the case of injection by the injector I, since the long plunger 30a is in a separated state, as it can be seen from FIGS. 5(A) and 5(B), a length of the combined pump head portion 10a and small diameter syringe 30 in the axial direction can be reduced greatly. As a result, operability is enhanced and manipulation of catheter treatment becomes easy.

Further, in this embodiment, the injecting system 100 is further provided with the outer peripheral temperature-maintaining heater units 10c, 10d mounted on an outer periphery of the small diameter syringe 30 and an outer periphery of the contrast medium bottle 70 suspended on a suspension stand. The outer peripheral temperature-maintaining heater unit 10c is connected to the heat source output terminal provided on the small size pump head portion 10a. Thus, as mentioned above, the power cord 10c2 for heat source can be connected to the heat source output terminal 20c in parallel with the connection of the plunger of the small diameter syringe 30 to the plunger connection portion 20b. Also, since the power cord 10c2 for heat source is connected to the heat source output terminal 20c of the pump head portion 10a, the power cord 10c2 for heat source does not obstruct manipulation of catheter treatment.

The heat source output terminals for the outer peripheral temperature-maintaining heater units 10c, 10d may be provided on both of the pump head portion 10a and the relay box 10h. In this case, heat source can be secured from the both of the pump head portion 10a and the relay box 10h.

As mentioned above, the temperatures of the outer periphery of the small diameter syringe 30 and the the outer periphery of the contrast medium bottle 70 can be maintained by the outer peripheral temperature-maintaining heater units 10c, 10d, thereby allowing the contrast medium to be maintained at a predetermined temperature. As a result, an injection pressure for injecting the contrast medium can be decreased, and at the same time, can be maintained constant. Further, since the contrast medium is not cooled during the catheter operation, treatment using a catheter can be conducted continuously, and even in the case where during treatment using a catheter, other manipulation is required and the catheter operation is once suspended, it is not necessary to heat the contrast medium again. Therefore, a treatment time can be shortened. It is noted that the predetermined temperature is not limited particularly, and may be, for example, 37° to 50°C. The predetermined temperature is preferably 40° to 50°C, further preferably 37° to 39°C.

The outer peripheral temperature-maintaining heater units 10c, 10d are not limited particularly as far as the contrast medium can be maintained at the predetermined temperature. The outer peripheral temperature-maintaining heater units 10c, 10d are preferably film heaters using heat generation obtained by energizing an electrically conductive film. Also, it is preferable that the outer peripheral temperature-maintaining heater units 10c, 10d have flexibility making it possible to be attached on the whole surfaces of the outer peripheries of the small diameter syringe 30 and the contrast medium bottle 70. As a result, winding and attaching on the small diameter syringe 30 is not bulky and does not obstruct catheter treatment.

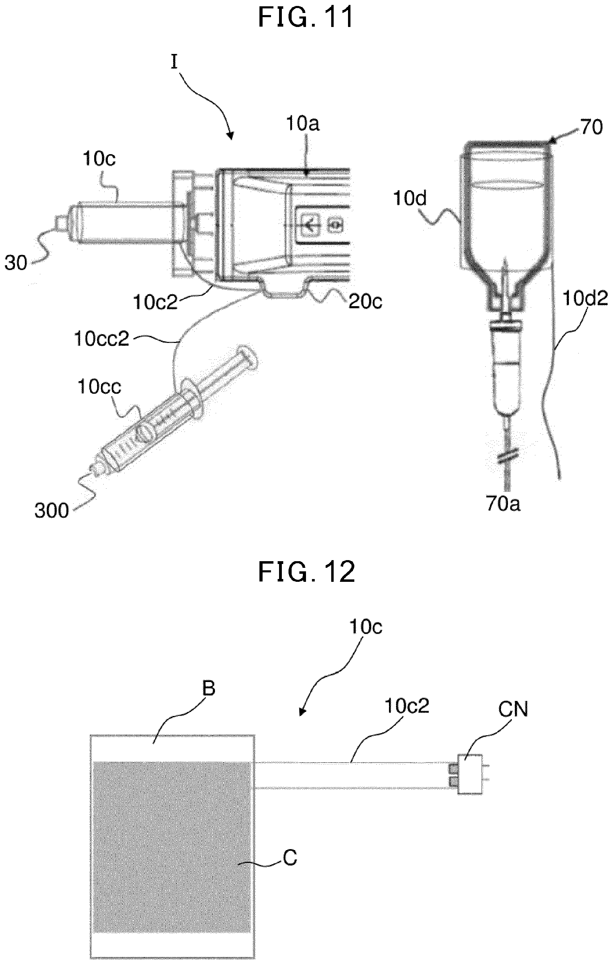

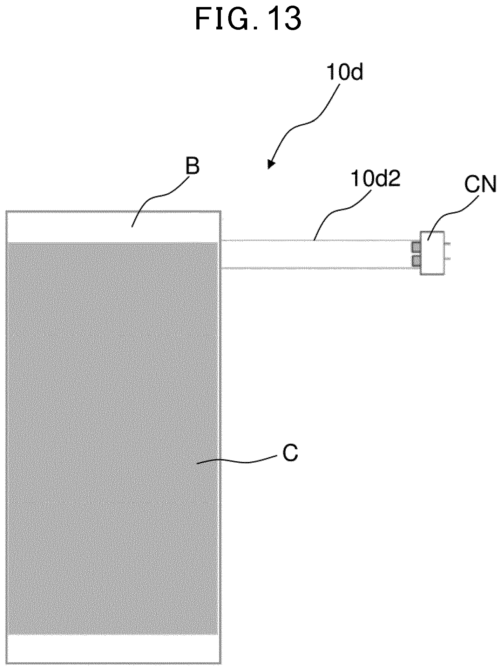

Also, it is preferable that the outer peripheral temperature-maintaining heater units 10c, 10d are film heaters having flexibility making it possible to be attached on the small diameter syringe 30 and the contrast medium bottle 70, and are formed from a transparent material in order to check the inside of the small diameter syringe 30 and the contrast medium bottle 70. In this case, it becomes easy to check a remaining amount of the contrast medium in the small diameter syringe 30 or the contrast medium bottle 70. As shown in FIG. 12 and FIG. 13, the outer peripheral temperature-maintaining heater units 10c, 10d may comprise, for example, a flexible substrate B provided with a flexible transparent electrically conductive film C, a connector CN being capable of connecting to the heat source output terminal 20c, and each of power cords 10c2, 10d2 for heat source. The sizes of the substrate B and the transparent electrically conductive film C can be changed appropriately depending on the size of the small diameter syringe 30 or the contrast medium bottle 70. For example, the size of the outer peripheral temperature-maintaining heater unit 10c for the small diameter syringe 30 can be 70 mm × 70 mm and a thickness thereof can be 0.2 mm, for example, in the case of a volume of the small diameter syringe 30 being 10 cc, and the size and the thickness thereof can be 70 mm × 100 mm and 0.2 mm, respectively in the case of a volume of the small diameter syringe 30 being 30 cc. Further, the size of the outer peripheral temperature-maintaining heater unit 10d for the contrast medium bottle 70 can be 85 mm × 185 mm and a thickness thereof can be 0.2 mm, for example, in the case of a volume of the contrast medium bottle 70 being 100 cc. An applied voltage of the outer peripheral temperature-maintaining heater unit 10c for the small diameter syringe 30 can be 1.5 to 3.0 V in the case of a volume of the small diameter syringe 30 being 10 cc, and can be 3.5 to 4.5 V in the case of a volume of the small diameter syringe 30 being 30 cc. Further, an applied voltage of the outer peripheral temperature-maintaining heater unit 10d for the contrast medium bottle 70 can be 5.0 to 7.0 V in the case of a volume of the contrast medium bottle 70 being 100 cc. For example, at an ambient temperature of 25° to 27°C, in the above-mentioned case of a volume of the small diameter syringe 30 being 10 cc, a target temperature of 37° to 50°C can be achieved within 300 seconds by applying a voltage of 1.5 V to 3.0 V to the outer peripheral temperature-maintaining heater unit 10c (for example, a size of 70 mm × 70 mm and a thickness of 0.2 mm) covering the whole small diameter syringe 30. Also, at an ambient temperature of 25° to 27°C, in the above-mentioned case of a volume of the small diameter syringe 30 being 30 cc, a target temperature of 37° to 50°C can be achieved within 300 seconds by applying a voltage of 3.5 V to 4.5 V to the outer peripheral temperature-maintaining heater unit 10c (for example, a size of 70 mm × 100 mm and a thickness of 0.2 mm). Also, at an ambient temperature of 25° to 27°C, in the above-mentioned case of a volume of the contrast medium bottle 70 being 100 cc, a target temperature of 37° to 50°C can be achieved within 300 seconds by applying a voltage of 5.0 V to 7.0 V to the outer peripheral temperature-maintaining heater unit 10d (for example, a size of 85 mm × 185 mm and a thickness of 0.2 mm) covering the whole contrast medium bottle 70. As mentioned above, the contrast medium in the vessel can be heated rapidly by applying a predetermined voltage to the outer peripheral temperature-maintaining heater unit. The outer peripheral temperature-maintaining heater units 10c, 10d may be attached to peripheries of the small diameter syringe 30 and the contrast medium bottle 70 using an adhesive double-coated tape so as to cover the peripheries thereof, or may be attached to peripheries of the small diameter syringe 30 and the contrast medium bottle 70, for example using a hook-and-loop fastener, so as to cover the peripheries thereof.

Further, the outer peripheral temperature-maintaining heater units 10c, 10d may be provided with a temperature sensor for monitoring an outer periphery temperature of the small diameter syringe 30 and an outer periphery temperature of the contrast medium bottle 70. A temperature of the contrast medium in the small diameter syringe 30 and the contrast medium bottle 70 can be controlled more accurately by providing the outer peripheral temperature-maintaining heater units 10c, 10d with the temperature sensor and controlling the outer periphery temperature of the small diameter syringe 30 and the contrast medium bottle 70 with the pump control device 10f. Therefore, an injection pressure can be kept constant. Thus, while decreasing an injection pressure of the contrast medium within a predetermined range, an injection rate can be increased and at the same time, deviation between the set values of an injection amount and an injection rate set with the pump control device 10f and the actual values of an injection amount and an injection rate of the contrast medium to be actually injected can be decreased greatly, thereby making highly accurate injection of the contrast medium possible. The pump control device 10f may control an injection amount and an injection rate in addition to the temperature of the contrast medium. In case that the temperature of the contrast medium, an injection amount of the contrast medium and an injection rate of the contrast medium can be controlled with the pump control device 10f, the contrast medium can be adjusted to a predetermined optimum temperature and can be injected in a desired amount at a desired injection rate. Further, the pump control device 10f may be configured so as to control the injection rate to be changed by every 0.1 ml/sec. In this case, the contrast medium can be injected according to a case of a disease of a patient and an affected part.

A method of use of the injecting system 100 is described below by referring to FIGS. 1 to 8. It is noted that the following method of use is only an example, and the injecting system 100 of the present invention is not limited to the following method of use.

First of all, in a state of the short plunger 30c and the long plunger 30a being combined with each other as shown in FIG. 3(A), the small diameter syringe 30 is connected to the contrast medium filling line 70a (see FIG. 4). Filling of the contrast medium into the small diameter syringe 30 and air vent operation are conducted through a manual push-pull operation by operating the long plunger 30a of the small diameter syringe 30.

Next, as shown in FIG. 3(B), the long plunger connecting portion 30b of the long plunger 30a is slid in a direction vertical to the axial direction of the small diameter syringe 30 to be separated from the short plunger 30c. After the long plunger 30a has been separated from the short plunger 30c, the small diameter syringe 30 is connected to the plunger connecting portion 20b to connect the pump head portion 10a to the small diameter syringe 30 as shown in FIG. 5(B).

Next, as shown in FIG. 4, the outer peripheral temperature-maintaining heater unit 10c for the small diameter syringe 30 and the outer peripheral temperature-maintaining heater units 10d for the contrast medium bottle 70 are attached to the small diameter syringe 30 and the contrast medium bottle 70, respectively, and the power cords 10c2, 10d2 are connected to the power source output terminal 20c of the pump head portion 10a and the power source output terminal of the relay box 10h, respectively to start temperature maintaining of the contrast medium.

Next, as shown in FIG. 2, the wing portion 80b of the microcatheter 80a for fixing the syringe is connected to the small diameter syringe 30.

Then, after optional injection setting suitable for a case of a disease has been made by means of the injection control section 10f, injection of the contrast medium is started by operating any of the hand switch portions 10b, 10g or the foot switch portion 10i.

It is noted that the above-mentioned injecting system 100 is not limited to the above-mentioned embodiment, and various modification or addition to the configurations or sizes based on the purport of the present invention can be made and are not excluded from the scope of the present invention.

### [Experimental Example]

An experimental example on an injection pressure for direct injection into the microcatheter (when the long size extended injection tube 51a (see FIG. 6(A)) is not used) which forms the basis of reduction of the injection pressure of the injecting system 100 of the present invention is described below in detail, using FIG. 8 and Tables 1 and 2.

FIG. 8 shows comparison of temperature transition of two bottles (about 180 ml) filled with hot water heated to 37°C. One bottle was wound with a film heater, and another bottle was not wound with a film heater.

Instruments used are 3-channel thermometer DATA LOGGER MTM-380SDJ, one wire temperature sensor TP-300 for measurement of an ambient temperature and two multi-purpose tape temperature sensors TS-04K (distal end of sensor: 4 mm wide × 100 mm long) for measurement of bottle temperature, all available from SATO SHOUJI INC.

As shown in FIG. 8, at an ambient temperature of 23°C, it is seen that while in the case of the bottle provided with no film heater, temperature thereof decreases up to a room temperature with a lapse of time, an initial temperature of 37°C is kept even 70 minutes after in the case of the bottle provided with the film heater. Therefore, it is seen that by use of the film heater (an outer peripheral temperature-maintaining heater unit), the temperature of the contrast medium can be kept at a predetermined temperature even after a lapse of time.

Table 1 shows a comparison data on injection pressures between the case of injection of the contrast medium at room temperature and the case of injection of the heated contrast medium when the injection rate is changed by every 0.1 ml/sec.

### <Example>

Injection pressures between the case where the contrast medium was heated and the case where the contrast medium was not heated (room temperature) with the microcatheter 80b being connected directly to the small diameter syringe 30 were compared. For heating the contrast medium, a film heater was wound on an outer periphery of the small diameter syringe, and the outer periphery temperature was measured with the temperature sensor used for measuring the temperature transition of FIG. 8 and the film heater was controlled so that the outer periphery of the small diameter syringe becomes a constant temperature. The small diameter syringe used, the contrast medium, temperature and injecting conditions are as follows.
Small size syringe: Inner diameter: 22.3 mm, volume: 30 cc
Contrast medium used: Iomeron 300: 300 mgI/ml
Catheter used: Estream, outer diameter of distal end: 0.68 mm/ outer diameter of proximal end: 0.97 mm, 135 cm
Setting of injection amount: Injection rate of 0.1 to 2.3 ml/sec
Total injection amount: 3 cc
Room temperature: 25°C
Heating device: A film heater is wound on and attached to an outer periphery of a small diameter syringe
Heating temperature: 37°C

The results are shown in Table 1.

**Table 1**

| Injection rate ml/sec | Room temperature injection pressure (PSI) | Injection pressure under heating (PSI) | Pressure differential (PSI) |
|---|---|---|---|
| 0.1 | unmeasurable | unmeasurable | - |
| 0.2 | 10 | 10 | 0 |
| 0.3 | 30 | 30 | 0 |
| 0.4 | 70 | 70 | 0 |
| 0.5 | 100 | 100 | 0 |
| 0.6 | 150 | 130 | -20 |
| 0.7 | 170 | 150 | -20 |
| 0.8 | 200 | 180 | -20 |
| 0.9 | 250 | 230 | -20 |
| 1.0 | 280 | 240 | -40 |
| 1.1 | 310 | 260 | -50 |
| 1.2 | 350 | 270 | -80 |
| 1.3 | 400 | 270 | -130 |
| 1.4 | 460 | 270 | -190 |
| 1.5 | 500 | 280 | -220 |
| 1.6 | 540 | 280 | -260 |
| 1.7 | 590 | 300 | -290 |
| 1.8 | 620 | 310 | -310 |
| 1.9 | 680 | 310 | -370 |
| 2.0 | 740 | 340 | -400 |
| 2.1 | 790 | 340 | -450 |
| 2.2 | 850 | 360 | -490 |
| 2.3 | Over | 370 | - |

As shown in Table 1, it is seen that the injection pressure under heating of the contrast medium is decreased significantly as compared with the injection of the contrast medium under room temperature condition, and the injection pressure difference especially in a region of a higher injection rate is large, namely 1/2 or less. Therefore, it is seen that an injection pressure can be decreased greatly by using a small diameter syringe and heating an outer periphery of the small diameter syringe as compared with a conventional injection device. It is seen that in particular when an injection rate is 1.3 to 2.3 ml/sec, a pressure differential becomes not less than 100 PSI and a significant effect can be obtained.

Further, Table 2 shows a comparison data between the injection pressures under heating at 37°C at the injection rate of 1.5 ml/sec, 1.8 ml/sec and 2.0 ml/sec and the injection pressures at an injection test example using a general large size injector (see page 4 of the package insert of Medical Device Approval Number: 21800BZZ10121000).

**Table 2**

| Injection rate /sec | General large size injector (PSI) | Small size injector (PSI) | Pressure differential |
|---|---|---|---|
| 1.5 | 600 | 280 | 320 |
| 1.8 | 800 | 310 | 490 |
| 2.0 | 1000 | 340 | 660 |

As shown in Table 2, it is seen that the injection pressure of the injecting system in Example of the present invention decreased significantly as compared with the general large size injector. It is noted that in Example of the present invention, the diameters of the catheter used were the outer diameter of distal end: 0.68 mm/ outer diameter of proximal end: 0.97 mm, and a length thereof was 135 cm, while in the catheter Masters used on the large size injector of the comparative example, the diameters of the catheter used were the outer diameter of distal end: 0.68 mm/ outer diameter of proximal end: 0.94 mm, and a length thereof was 125 cm. The injector was ZMA700 model A (Sheen Man Co., Ltd). As shown in Table 2, even under the disadvantageous condition of the length being longer by 10 cm in Example of the present invention, it is seen that a very significant effect such that the difference in the injection pressure is half or more as compared with the catheter having nearly the same size.

### <Second embodiment>

Second embodiment shows an example where an outer periphery temperature-maintaining heater unit is applied to a syringe for manual operation, in which a suspension including an anticancer drug and a contrast medium used for treatment of embolus is filled. Hereinbelow, explanation common to the first embodiment is omitted, and differential points are mainly described. It is noted that each configuration described in the second embodiment can be applied to the first embodiment, and each configuration described in the first embodiment can be applied to the second embodiment.

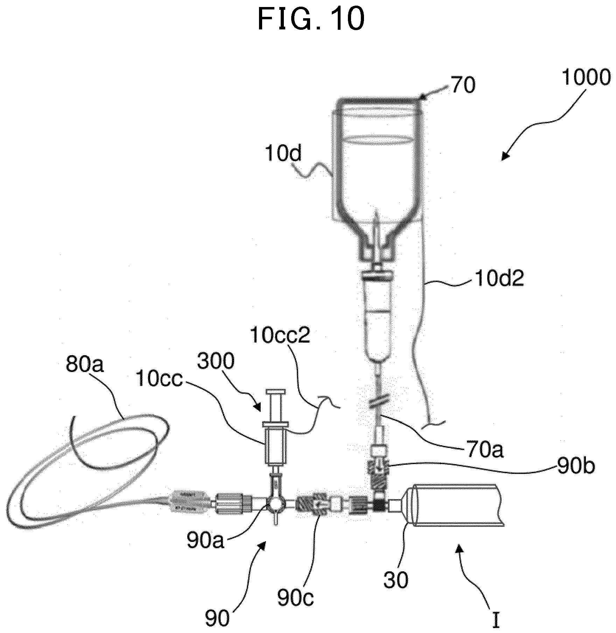

The system 1000 for temperature-maintaining and injecting a therapeutic suspension medicine for a microcatheter of this embodiment comprises, as shown in FIG. 10, the microcatheter 80a, the syringe 300 for manual operation, in which a suspension including an anticancer drug and a contrast medium used for treatment of embolus are filled, and the connecting portion 90 for connecting the syringe 300 for manual operation and the microcatheter 80a. Further, on the outer periphery of the syringe 300 for manual operation is mounted the outer peripheral temperature-maintaining heater unit 10cc as shown in FIG. 10 and FIG. 11.

In this embodiment, as shown in FIG. 10 and FIG. 11, the connecting portion 90 is mounted to the injector I and the small diameter syringe 30 attached to the injector I and the syringe 300 for manual operation can be connected to the connecting portion 90. It is noted that the injector I and the small diameter syringe 30 are not limited to those used in the first embodiment, and other structures may be used. Known syringes for manual operation can be used as the syringe 300 for manual operation.

The connecting portion 90 is not limited to the structure shown in the drawing as far as the syringe 300 for manual operation can be connected thereto. In this embodiment, the connecting portion 90 has a three-way cock 90a for switching a flow path between a flow path from the syringe 300 for manual operation to the microcatheter 80a and a flow path from the small diameter syringe 30 to the microcatheter 80a, a one-way valve 90b which is connected to the contrast medium filling line 70a connected to the contrast medium bottle 70 and is opened when filling the contrast medium into the small diameter syringe 30 and is closed when injecting the contrast medium, and a one-way valve 90c which is disposed between the small diameter syringe 30 and the three-way cock 90a and is closed when filling the contrast medium and is opened when injecting the contrast medium. Switching these valves enables filling of the contrast medium, automatic injection of the contrast medium and manual injection of a suspension used for treatment of embolus to be switched.

The outer peripheral temperature-maintaining heater unit 10cc mounted on the outer periphery of the syringe 300 for manual operation is heated by connecting a cord 10cc2 for heat source to a power source output terminal. The power source output terminal may be disposed on any device, and in this embodiment, as shown in FIG. 11, the cord 10cc for heat source is connected to the heat source output terminal 20c provided on the pump head portion 10a. In this case, for example, two heat source output terminals may be provided on the pump head portion 10a. When the outer peripheral temperature-maintaining heater unit 10cc of the syringe 300 for manual operation is connected to the heat source output terminal 20c provided on the pump head portion 10a, the power cord 10cc2 for heat source does not obstruct manipulation of catheter treatment, and operations from imaging of a site to be treated to treatment of embolus can be carried out in the same visual field.

According to this embodiment, as mentioned above, the outer peripheral temperature-maintaining heater unit 10cc is mounted on the outer periphery of the syringe 300 for manual operation as shown in FIG. 10 and FIG. 11. Thus, the temperature of the outer periphery of the syringe 300 for manual operation can be maintained and the suspension can be kept at a predetermined temperature in the same manner as in the outer peripheral temperature-maintaining heater unit 10c mounted on the small diameter syringe 30 described in the first embodiment. As a result, reduction of an injection pressure due to decrease of a viscosity when injecting the suspension can be achieved and the injection pressure can be kept constant. It is noted that the predetermined temperature is not limited particularly, and for example, can be 37° to 50°C. The predetermined temperature is preferably 40° to 50°C, further preferably 37° to 39°C. For example, at an ambient temperature of 25° to 27°C, in the above-mentioned case of a volume of the syringe 300 being 10 cc, a target temperature of 37° to 50°C can be achieved within 300 seconds by applying a voltage of 1.5 V to 3.0 V to the outer peripheral temperature-maintaining heater unit 10cc (for example, a size of 70 mm × 70 mm and a thickness of 0.2 mm) covering the whole syringe 300. Also, at an ambient temperature of 25° to 27°C, in the above-mentioned case of a volume of the syringe 300 being 30 cc, a target temperature of 37° to 50°C can be achieved within 300 seconds by applying a voltage of 3.5 V to 4.5 V to the outer peripheral temperature-maintaining heater unit 10cc (for example, a size of 70 mm × 100 mm and a thickness of 0.2 mm). Thus, by applying a predetermined voltage to the outer peripheral temperature-maintaining heater unit, the suspension medicine in the syringe can be heated rapidly.

In the treatment of embolus, the suspension medicine is injected by the syringe 300 for manual operation to a plurality of tumors of a patient or a plurality of nutrition blood vessels extending to the plurality of the tumors. It takes usually about 2 to 3 hours in total to complete this treatment, and the injection of the suspension medicine is conducted to plural sites at predetermined intervals. Therefore, though very complicated operations need be repeated in a clean operating field and unclean operating field in a conventional heating method, according to this embodiment, basically no complicated operation is necessary and a treating time can be made shorter. This point is described below in detail.

In a conventional treatment of embolus, as shown in Non-Patent Document 2, a suspension medicine was heated using a water bath. The conventional treatment of embolus is carried out as follows:
(1) Water (2 litters) is poured in a water bath and a power source is turned on.
(2) Proper amounts of anticancer drug and contrast medium are sucked with a syringe and poured into a mixing vessel.
(3) An operator moves to a location of the water bath.
(4) The mixing vessel is put in a test tube rack, which is then put in the water bath.
(5) The operator returns to an operating table.
(6) The operator moves to the location of the water bath.
(7) Immediately before use, the mixing vessel is taken out and every 1 ml is dispensed and injected with a syringe.
(8) The operator attaches and removes a needle to/from the syringe when sucking a mixture and mounting a microcatheter.

In a conventional heating method using a water bath, in addition to the above-mentioned complicated operations, once the suspension medicine is taken out of the water bath, the suspension medicine is cooled from the time when the suspension medicine is injected at an embolus site to the time when the suspension medicine is injected at a next embolus site, and therefore, it is necessary to keep the suspension medicine in the water bath and repeat operation for taking out a necessary amount as required. Meanwhile, according to this embodiment, even if a necessary amount for embolus at a plurality of embolus sites is filled in the syringe 300 for manual operation, since the suspension medicine is continuously heated by the outer peripheral temperature-maintaining heater unit 10cc, reduction of an injection pressure is maintained and easy injection is possible and in addition, continuous administration to a plurality of embolus points is possible. Therefore, no complicated operation like a conventional method is required, and an injection pressure is reduced, injection is easy and a treatment time can be reduced.

As mentioned above, the system for temperature-maintaining and injecting a contrast medium for a microcatheter according to embodiment 1 comprises an injector for injecting the contrast medium and a pump control device connected to the injector, wherein the injector for injecting the contrast medium comprises a pump head portion and a small diameter syringe connected to the pump head portion, wherein the system for temperature-maintaining and injecting the contrast medium further comprises an outer peripheral temperature-maintaining heater unit to be attached on an outer periphery of the small diameter syringe, and wherein the outer peripheral temperature-maintaining heater unit to be attached on the outer periphery of the small diameter syringe is connected to a heat source output terminal provided on the pump head portion..

According to the configuration of the above-mentioned embodiment 1, the temperature of the outer periphery of the small diameter syringe can be maintained by the outer peripheral temperature-maintaining heater unit, and the contrast medium can be kept at a predetermined temperature. Thus, an injection pressure of the contrast medium when injecting it can be reduced and the injection pressure can be kept constant. Further, since the contrast medium is not cooled during a catheter operation, treatment using a catheter can be carried out continuously, and even if during treatment using a catheter, other manipulation is required and the catheter operation is once interrupted, it is not necessary to warm the contrast medium again. Therefore, a treatment time can be shortened.

Further, in embodiment 2, the system for temperature-maintaining and injecting the contrast medium of embodiment 1, further comprises an arm stand to which the pump head portion mounted, and the arm stand being capable of moving the pump head portion to an operating field.

According to the configuration of the above-mentioned embodiment 2, it is possible to inject the contrast medium directly to the microcatheter, and due to disuse or significant shortening of the extended injection tube, further reduction of an injection pressure and decrease in cost (the extended injection tube is unnecessary or is shortened and a loss of the contrast medium is eliminated) can be achieved.

Further, in embodiment 3, the system for temperature-maintaining and injecting the contrast medium of embodiment 1 or 2, wherein the pump control device is capable of controlling a temperature of the contrast medium, an injection amount of the contrast medium and an injection rate of the contrast medium.

According to the configuration of the above-mentioned embodiment 3, since a temperature of the contrast medium, an injection amount of the contrast medium and an injection rate of the contrast medium can be controlled by the pump control device, the contrast medium can be adjusted to an optimum temperature, and can be injected in a desired injection amount at a desired injection rate.

Further, in embodiment 4, the system for temperature-maintaining and injecting the contrast medium of any one of embodiments 1 to 3, wherein the pump head portion is connected to a drive unit for operating the pump head portion through a flexible shaft, and is remotely driven by a driving force of the drive unit.

According to the configuration of the above-mentioned embodiment 4, since no motor drive unit is required on the pump head portion, the pump head portion can be made more lighter and small. As a result, it is possible to move the pump head portion smoothly to an operating field by a more compact arm stand.

Further, in embodiment 5, the system for temperature-maintaining and injecting the contrast medium of any one of claims 1 to 4, wherein a volume of the small diameter syringe is 30 to 50 cc and an inner diameter of the small diameter syringe is 20 to 30 mm.

According to the configuration of the above-mentioned embodiment 5, the volume and the inner diameter of the syringe are small, and the syringe can be used suitably on the above-mentioned pump head portion, thereby achieving reduction of an injection pressure and enhancing operability of the arm stand while moving the pump head portion.

Further, in embodiment 6, the system for temperature-maintaining and injecting the contrast medium of any one of claims 1 to 5, wherein the volume of the small diameter syringe is set 30cc, which is calculated from a maximum injection amount for one dose of the contrast medium in microcatheter treatment, wherein the small diameter syringe comprises a short plunger connected to a gasket of the small diameter syringe and a long plunger connected to the short plunger, wherein the small diameter syringe can be changed over to a connected state in which the long plunger is connected to the short plunger when manually filling the contrast medium to the small diameter syringe or to a separated state in which the long plunger is separated from the short plunger when connecting the small diameter syringe to the pump head portion.

According to the configuration of the above-mentioned embodiment 6, by calculating a maximum injection amount of one dose of the contrast medium in microcatheter treatment, a necessary minimum syringe size of 30 cc is obtained, and a minimum plunger (short plunger) necessary for the injection and filling thereof is provided. In this case, in addition to miniaturization of the syringe and a smaller diameter of the syringe, due to a synergistic effect of a heat retaining property by the outer peripheral temperature-maintaining heater unit and good mobility of the arm stand and the small size pump head portion, it is possible to enhance a accuracy of the injection amount of the contrast medium and reduce an injection pressure. Further, according to the above-mentioned configuration, high accuracy and high rate injection of microdose aiming at reducing excessive high pressure injection can be achieved by a simple operation at the time of injection. Also, according to the above-mentioned configuration, large improvement of the control device and the like is not necessary, and by a light weight and downsizing of the pump head portion of the injector, simple and effective high accuracy and high rate injection of microdose becomes possible while making use of portability, which is extremely advantageous for use of a microcatheter at its operating site. Further, by combination of embodiment 3 and embodiment 5, the pump head portion becomes smaller and mobility thereof becomes satisfactory in the case where the pump head portion is a small size portable type by the lightest configuration with a remote drive method (an external motor) using a drive source from the outside.

Further, contrary requirements demanded for the small diameter syringe in operating the plunger (a longer plunger is preferable when filling manually while a shorter plunger is preferable when filling automatically or moving the pump head portion) can be satisfied by using a two-stage separable syringe.

Further, in embodiment 7, the system for temperature-maintaining and injecting the contrast medium of any one of embodiments 1 to 6, wherein the outer peripheral temperature-maintaining heater unit is a film heater using heat generation obtained by energizing an electrically conductive film, wherein the film heater has flexibility enabling the film heater to be attached to the whole surface of an outer periphery of the small diameter syringe and a contrast medium bottle, and is formed from a transparent material in order to check inside of the small diameter syringe and the contrast medium bottle.

According to the configuration of the above-mentioned embodiment 7, while a small size and lighter weight of the small diameter syringe are maintained, the contrast medium can be maintained at a predetermined temperature, and the inside of the syringe and the bottle can be checked through the film heater formed from the transparent material.

Further, in embodiment 8, the system for temperature-maintaining and injecting the contrast medium of any one of embodiments 1 to 7, further comprising a contrast medium bottle containing a contrast medium to be filled in the small diameter syringe, wherein the system of temperature-maintaining and injecting the contrast medium includes a second outer peripheral temperature-maintaining heater unit being mounted on an outer periphery of the contrast medium bottle.

According to the configuration of the above-mentioned embodiment 8, since not only the small diameter syringe but also the outer periphery of the contrast medium bottle is heated, a cooled contrast medium is not supplied to the small diameter syringe. Therefore, heating of the contrast medium can be accelerated and a temperature of the contrast medium to be supplied becomes stable.

Further, in embodiment 9, the system for temperature-maintaining and injecting the contrast medium of claim 8, wherein each of the outer peripheral temperature-maintaining heater unit and the second outer peripheral temperature-maintaining heater unit comprises a temperature sensor monitoring each of an outer periphery temperature of the small diameter syringe and an outer periphery temperature of the contrast medium bottle, and the outer periphery temperatures of the small diameter syringe and the contrast medium bottle are controlled to a predetermined temperature.

According to the configuration of the above-mentioned embodiment 9, temperatures of the contrast medium in the small diameter syringe and the contrast medium bottle can be controlled more accurately. Therefore, an injection pressure can be maintained constant. As a result, while reducing the injection pressure of the contrast medium within a predetermined range, an injection rate can be increased, and in addition, deviations of the injection amount and the injection rate of the actually injected contrast medium from the injection amount and the injection rate which are set by the pump control device can be reduced greatly, and highly accurate injection of the contrast medium can be achieved.

Further, in embodiment 10, the system for temperature-maintaining and injecting the contrast medium of any one of claims 1 to 9, further comprising a microcatheter, a syringe for manual operation for containing a suspension including an anticancer drug and a contrast medium for treatment of embolus, and a connecting portion for connecting the syringe for manual operation and the microcatheter, wherein the connecting portion is mounted at an end of the small diameter syringe, wherein the system for temperature-maintaining and injecting the contrast medium includes a third outer peripheral temperature-maintaining heater unit being mounted on an outer periphery of the syringe for manual operation.

According to the configuration of the above-mentioned embodiment 10, even if an amount of the suspension medicine necessary for embolus at a plurality of parts is collectively filled in the syringe for manual operation, by heating the suspension medicine continuously by the outer peripheral temperature-maintaining heater unit, reduction of the injection pressure is maintained and the injection can be carried out easily, and in addition, the suspension medicine can be administrated continuously for embolus at a plurality of parts. Therefore, no complicated operations need to be conducted like a conventional method, and it is possible to reduce an injection pressure, conduct the injection easily and shorten a treatment time.

Further, in embodiment 11, a system for temperature-maintaining and injecting a therapeutic suspension medicine for a microcatheter comprises a microcatheter, a syringe for manual operation for containing a suspension including an anticancer drug and a contrast medium for treatment of embolus, and a connecting portion for connecting the syringe for manual operation and the microcatheter, wherein the system for temperature-maintaining and injecting a therapeutic suspension medicine further comprises an outer peripheral temperature-maintaining heater unit being mounted on an outer periphery of the syringe for manual operation.

According to the configuration of the above-mentioned embodiment 11, even if an amount of the suspension medicine necessary for embolus at a plurality of parts is filled in the syringe for manual operation, by heating the suspension medicine continuously by the outer peripheral temperature-maintaining heater unit, reduction of the injection pressure is maintained and the injection can be carried out easily, and in addition, the suspension medicine can be administrated continuously for embolus at a plurality of parts. Therefore, no complicated operations need to be conducted like a conventional method, and it is possible to reduce an injection pressure, conduct the injection easily and shorten a treatment time.

Further, in embodiment 12, the system for temperature-maintaining and injecting a therapeutic suspension medicine of embodiment 11, wherein the outer peripheral temperature-maintaining heater unit is a film heater using heat generation obtained by energizing an electrically conductive film, wherein the film heater has flexibility enabling the film heater to be attached to the whole outer peripheral parts of the syringe for manual operation, and is formed from a transparent material in order to check inside of the syringe for manual operation.

According to the configuration of the above-mentioned embodiment 12, while a small size and lighter weight of the syringe for manual operation are maintained, the suspension medicine can be maintained at a predetermined temperature, and the inside of the syringe can be checked easily through the film heater formed from the transparent material.

Further, in embodiment 13, the system for temperature-maintaining and injecting a therapeutic suspension medicine of claim 11 or 12, wherein the outer peripheral temperature-maintaining heater unit comprises a temperature sensor for monitoring an outer periphery temperature of the syringe for manual operation, and the outer periphery temperature of the syringe for manual operation is controlled at a predetermined temperature.

According to the configuration of the above-mentioned embodiment 13, temperature of the suspension medicine in the syringe for manual operation can be controlled more accurately. Therefore, an injection pressure can be maintained constant. As a result, while reducing the injection pressure of the suspension medicine within a predetermined range, a sense of syringe operation does not change.

Further, each of the above-mentioned injecting systems relates to in particular a recent social issue on refugees involved in treatment of a cancer, and there is an increasing possibility of using the system for treatment of embolus (cutting off a nutrition source for a cancer by feeding an embolic substance from a microcatheter) which is expected by cancer refugees as a countermeasure for solving the problem, the system can be produced relatively easily since its structure is simple, and in use of the system, improvement of functionality (reduction of an injection pressure and high accuracy of an injection amount) and labor saving (reduction of a loss of a contrast medium and saving in cost of an extension tube for injection) are made. Thus, there is much expectation for practical use of the system since it is considered that the system can be used satisfactorily from the viewpoint of cost performance and there is no problem with safety.

### Explanation of Symbols

- 100: System for temperature-maintaining and injecting contrast medium
- 1000: System for temperature-maintaining and injecting suspension
- 10a: Pump head portion
- 10b: Hand switch portion at an operating field side
- 10c: Outer peripheral temperature-maintaining heater unit for a small diameter syringe
- 10cc: Outer peripheral temperature-maintaining heater unit of a small diameter syringe for manual operation
- 10c2: Power cord for heat source
- 10d: Outer peripheral temperature-maintaining heater unit for a contrast medium bottle
- 10d2: Power cord for heat source
- 10e: Motor box
- 10f: Injection control section (pump control device)
- 10g: Hand switch portion at an operation room side
- 10h: Relay box
- 10i: Foot switch portion at an operating field side
- 10j: Monitor
- 10k: Main unit
- 10l, 10m, 10n, 10o: Cable
- 20a: Syringe mounting portion
- 20b: Plunger connecting portion
- 20c: Heat source output terminal
- 20d: Housing
- 30: Small diameter syringe
- 300: Syringe for manual operation
- 30a: Long plunger
- 30b: Long plunger connecting portion
- 30c: Short plunger
- 40: Plunger
- 51: Fixed pole
- 51a: Long size extended injection tube
- 52: Arm stand
- 52a: Short size extended injection tube
- 61: Motor drive unit
- 62: Plunger drive unit
- 63: Flexible shaft
- 70: Contrast medium bottle
- 70a: Contrast medium filling line
- 80a: Microcatheter
- 80b: Wing portion for fixing a syringe
- 90: Connecting portion
- 90a: Three-way cock
- 90b, 90c: One-way valve
- B: Substrate
- C: Transparent electrically conductive film
- CN: Connector
- I: Injector
- S1: Injection operation switch
- S2: Filling operation switch
- SB: Safety button
- T1: Temperature of the outer periphery of the contrast medium bottle in an elapsed time when using the outer peripheral temperature-maintaining heater unit
- T2: Temperature of the outer periphery of the contrast medium bottle in an elapsed time when using no outer peripheral temperature-maintaining heater unit
- T3: Ambient temperature
- X: Catheter operating table

## Claims

1. A system for temperature-maintaining and injecting a contrast medium for a microcatheter comprising an injector for injecting the contrast medium and a pump control device connected to the injector,
wherein the injector for injecting the contrast medium comprises a pump head portion and a small diameter syringe connected to the pump head portion,
wherein the system for temperature-maintaining and injecting the contrast medium further comprises an outer peripheral temperature-maintaining heater unit to be attached on an outer periphery of the small diameter syringe, and
wherein the outer peripheral temperature-maintaining heater unit to be attached on the outer periphery of the small diameter syringe is connected to a heat source output terminal provided on the pump head portion.

2. The system for temperature-maintaining and injecting the contrast medium of claim 1, further comprising an arm stand to which the pump head portion mounted, and the arm stand being capable of moving the pump head portion to an operating field.

3. The system for temperature-maintaining and injecting the contrast medium of claim 1 or 2, wherein the pump control device is capable of controlling a temperature of the contrast medium, an injection amount of the contrast medium and an injection rate of the contrast medium.

4. The system for temperature-maintaining and injecting the contrast medium of any one of claims 1 to 3, wherein the pump head portion is connected to a drive unit for operating the pump head portion through a flexible shaft, and is remotely driven by a driving force of the drive unit.

5. The system for temperature-maintaining and injecting the contrast medium of any one of claims 1 to 4, wherein a volume of the small diameter syringe is 30 to 50 cc and an inner diameter of the small diameter syringe is 20 to 30 mm.

6. The system for temperature-maintaining and injecting the contrast medium of any one of claims 1 to 5, wherein the volume of the small diameter syringe is set 30cc, which is calculated from a maximum injection amount for one dose of the contrast medium in microcatheter treatment,
wherein the small diameter syringe comprises a short plunger connected to a gasket of the small diameter syringe and a long plunger connected to the short plunger,
wherein the small diameter syringe can be changed over to a connected state in which the long plunger is connected to the short plunger when manually filling the contrast medium to the small diameter syringe or to a separated state in which the long plunger is separated from the short plunger when connecting the small diameter syringe to the pump head portion.

7. The system for temperature-maintaining and injecting the contrast medium of any one of claims 1 to 6, wherein the outer peripheral temperature-maintaining heater unit is a film heater using heat generation obtained by energizing an electrically conductive film, wherein the film heater has flexibility enabling the film heater to be attached to the whole surface of an outer periphery of the small diameter syringe and a contrast medium bottle, and is formed from a transparent material in order to check inside of the small diameter syringe and the contrast medium bottle.

8. The system for temperature-maintaining and injecting the contrast medium of any one of claims 1 to 7, further comprising a contrast medium bottle containing a contrast medium to be filled in the small diameter syringe, wherein the system of temperature-maintaining and injecting the contrast medium includes a second outer peripheral temperature-maintaining heater unit being mounted on an outer periphery of the contrast medium bottle.

9. The system for temperature-maintaining and injecting the contrast medium of claim 8, wherein each of the outer peripheral temperature-maintaining heater unit and the second outer peripheral temperature-maintaining heater unit comprises a temperature sensor monitoring each of an outer periphery temperature of the small diameter syringe and an outer periphery temperature of the contrast medium bottle, and the outer periphery temperatures of the small diameter syringe and the contrast medium bottle are controlled to a predetermined temperature.

10. The system for temperature-maintaining and injecting the contrast medium of any one of claims 1 to 9, further comprising a microcatheter, a syringe for manual operation for containing a suspension including an anticancer drug and a contrast medium for treatment of embolus, and a connecting portion for connecting the syringe for manual operation and the microcatheter, wherein the connecting portion is mounted at an end of the small diameter syringe,
wherein the system for temperature-maintaining and injecting the contrast medium includes a third outer peripheral temperature-maintaining heater unit being mounted on an outer periphery of the syringe for manual operation.

11. A system for temperature-maintaining and injecting a therapeutic suspension medicine for a microcatheter comprising a microcatheter, a syringe for manual operation for containing a suspension including an anticancer drug and a contrast medium for treatment of embolus, and a connecting portion for connecting the syringe for manual operation and the microcatheter, wherein the system for temperature-maintaining and injecting a therapeutic suspension medicine further comprises an outer peripheral temperature-maintaining heater unit being mounted on an outer periphery of the syringe for manual operation.

12. The system for temperature-maintaining and injecting a therapeutic suspension medicine of claim 11, wherein the outer peripheral temperature-maintaining heater unit is a film heater using heat generation obtained by energizing an electrically conductive film, wherein the film heater has flexibility enabling the film heater to be attached to the whole outer peripheral parts of the syringe for manual operation, and is formed from a transparent material in order to check inside of the syringe for manual operation.

13. The system for temperature-maintaining and injecting a therapeutic suspension medicine of claim 11 or 12, wherein the outer peripheral temperature-maintaining heater unit comprises a temperature sensor for monitoring an outer periphery temperature of the syringe for manual operation, and the outer periphery temperature of the syringe for manual operation is controlled at a predetermined temperature.
